# EUROPÄISCHE PATENTSCHRIFT

(11) **EP 0 595 012 B1**
(45) Veröffentlichungstag und Bekanntmachung des Hinweises auf die Patenterteilung: **11.06.1997**
(21) Anmeldenummer: 93115004.9
(22) Anmeldetag: 17.09.1993
(51) Int. Cl.: A61K 7/48

(54) **Kosmetische Mittel**
Cosmetic composition
Composition cosmétique

(30) Priorität: 21.09.1992 DE 4231544
(43) Veröffentlichungstag der Anmeldung: 04.05.1994
(73) Patentinhaber: Chemisches Laboratorium Dr. Kurt Richter GmbH, D-12159 Berlin (DE)
(72) Erfinder: Petersen, Rolf-Dieter Dr., D-14199 Berlin (DE)
(74) Vertreter: VOSSIUS & PARTNER

(56) Entgegenhaltungen:
- EP-A- 0 286 869
- EP-A- 0 404 661
- DE-A- 4 032 972
- US-A- 5 158 772

## Beschreibung

Die Erfindung betrifft kosmetische Mittel zum Schutz der Haut, insbesondere zur Vorbeugung von Entzündungen der Haut. Die erfindungsgemäßen Mittel enthalten Zellwandfraktionen gram-positiver Eubakterien. Bevorzugt sind gram-positive Eubakterien, deren Basenzusammensetzung der DNS einen hohen Guanin- und Cytosinanteil (G+C Mol % : größer 50 %) aufweist, insbesondere der Bakterienspezies Micrococcus luteus.

Die Bedeutung von UV-Absorbern und bestimmten Antioxydantien, die Hautschäden und der beschleunigten, lichtbedingten Hautalterung vorbeugen, für den Lichtschutz in der Kosmetik ist seit längerer Zeit bekannt. Jüngste Fortschritte der Photochemie mit Erkenntnissen bezüglich inflammatorischer Prozesse auf zellulärer Ebene zeigen auf, daß der lichtbedingten Hautalterung durch topischen Einsatz bestimmter Substanzen wirkungsvoll vorgebeugt werden kann. Epidermis-Zellen reagieren auf UV-Strahlen in vielfältiger Weise, was man als "inflammmatory response" bezeichnet. Eine lokale und systemische Entzündungsreaktion nach UVB-Bestrahlung wurde beschrieben. Ausgelöst wurde die Reaktion durch die Freisetzung von Zytokinen wie Interleukin 1 und 6 (IL-1, IL-6), Tumornekrose-Faktor α (TNF-α) u.a. Eine signifikante Abnahme in der Ausschleusung von IL-6 nach Applikation von Corticosteroiden als anti-entzündliches Agens wurde beobachtet.

Daneben können Entzündungsreaktionen nicht nur von UVB-sondern auch von UVA-Bestrahlung und PUVA-Behandlung (Psoralen plus UVA) ausgelöst werden. Freie Radikale führen zur Peroxidation von Zellmembran-Lipiden. Durch Phospholipase-Aktivierung wird Arachidonsäure freigesetzt, die einerseits über den Cyclooxygenase-Weg in Prostaglandine, Thromboxane und Prostacyclin und andererseits über den Lipoxygenase-Weg in Leukotriene und verschiedene mono-, di- und trihydroxylierte Eicosantetraensäuren (HETE's) metabolisiert.

Die durch Arachidonsäure-Freisetzung eingeleitete Entzündungsreaktion reduziert gleichzeitig die Aktivität der antioxydativ wirkenden Enzyme Katalase und Superoxid Dismutase (SOD). Das geschieht nicht nur nach UVB-Einwirkung, sondern auch nach UVA-Bestrahlung.

Irritations- bzw. Sensibilierungs-Potentiale von Inhaltstoffen kosmetischer oder pharmazeutischer Präparate als mögliche Auslöser von Entzündungsreaktionen sind ebenfalls als Faktoren beschleunigter Kautalterung bekannt.

Daneben können UV-Strahlen direkt Reaktionen katalysieren, ohne daß diese durch Entzündungsprozesse ausgelöst werden. Die durch die helikale Konformation der DNS benachbarten Thymin-Bausteine eines Stranges dimerisieren unter Lichteinfluß. Ein zellendogenes enzymatisches Reparatursystem kann diesen Schaden erkennen, das Segment mit dem Dimer heraustrennen und nach Neusynthese korrekt ersetzen (Excisionsreparatur). Ist dieser Reparaturprozeß altersbedingt reduziert oder durch zu starke Strahlung überfordert, kann dieses Thymin-Dimer durch Verzerrung der räumlichen Anordnung der DNS Ursache für Mutationen sein. Kosmetische Mittel mit inaktivierten Kulturen aus Bifidobakterien, die den natürlichen DNS-Repair-Prozeß fördern, sind in EP-A-43128 beschrieben.

Unter den vielfältigen Möglichkeiten der lichtbedingten Schadensauslösung mit der Folge der beschleunigten Hautalterung haben Entzündungsreaktionen auf zellulärer Ebene eine besondere Bedeutung. Entzündungen, ausgelöst durch UV-Bestrahlung, Irritantien oder toxische Agentien, setzen diverse Mediatoren frei, wie Histamin, Prostaglandine oder Leukotriene. Verbunden damit sind epidermale Zellschäden (sunburn cells), Bildung apoptotischer Zellen, DNS-Schäden, die zur Bildung maligner Melanome oder von Basalzellen-Karzinomen führen.

Daneben laufen degenerative Prozesse ab, wie die aktinische Degeneration: Elastische Fasern wandeln sich um zu verklumptem amorphen Material; Kollagenfibrillen verlieren Elastizität durch Quervernetzung.

Besonders kritisch sind länger anhaltende Reize, die zu chronischen Entzündungen führen. Die erzwungene Neubildung der Haut wird durch eine erhöhte Zellteilung erreicht. Da bei der komplizierten Zellteilung aber das Reparatursystem weitgehend lahmgelegt ist, kommt es bei anhaltendem Reizeinfluß zu Schäden an der DNS und damit zu Veränderungen der Erbanlagen.

Diese Zusammenhänge belegen die Bedeutung von Stoffen zur Vorbeugung von Entzündungen der Haut.

Entzündungsprozesse der Haut können beispielsweise durch topische Anwendung von Corticosteroid- oder Acetylsalicylsäure-Präparaten behandelt werden. Da diese Stoffe nach Resorption über den Kreislauf im ganzen Körper verteilt werden, spricht man von einer systemischen Wirkung. Stoffe mit systemischer Wirkung sind für den kosmetischen Einsatz nicht zugelassen, da eine Vielzahl unerwünschter Nebenreaktionen möglich ist.

Fraktionen aus Pflanzenextrakten mit einer antientzündlichen Aktivität sind bekannt.

Als aktiv haben sich im besonderen Substanzen erwiesen, die am Aufbau von Zellwänden beteiligt sind.

Während die Pflanzenzellwand hauptsächlich aus Polysacchariden und Lignin besteht, ist der Aufbau der Zellwand z.B. bei gram-positiven Bakterien erheblich differenzierter. In Zellwänden gram-positiver Bakterien finden sich nämlich neben Polysacchariden vielfältig strukturierte Verbindungen, wie Peptidoglycane (Mureine) und "accessory polymers" wie Teichonsäuren, Teichuronsäuren oder andere amphiphile Makromoleküle.

Somit läßt bei gram-positiven Bakterien die Vielfalt biologischer Zellwandbestandteile ein größeres Potential zur Gewinnung von Fraktionen mit spezifischen Eigenschaften zu.

Es wurden bereits unterschiedliche biologische Aktivitäten von Zellwandbestandteilen verschiedener Mikroorganismen beschrieben.

Beispielsweise wird in der EP 0 201 332 eine antiinfektiöse Wirkung bei Darminfektionen durch Polysaccharide aus Zellwänden von Bifidobakterien beschrieben.

Diese biologisch aktiven Fraktionen von Mikroorganismen betreffen aufgrund Ihrer Indikation das pharmazeutische Anwendungsgebiet.

Der Erfindung liegt die Aufgabe zugrunde, kosmetische Mittel zum Schutz der Haut, insbesondere zur Vorbeugung von Entzündungen der Haut, zur Verfügung zu stellen, die einen wichtigen Beitrag zum Schutz der Haut gegen beschleunigte Alterung durch die Einwirkung von UV-Licht oder Irritantien leisten können.

Es wurde überraschend gefunden, daß Zellwandfraktionen von gram-positiven Eubakterien eine hohe vorbeugende Wirkung gegenüber Entzündungen der Haut besitzen. Bevorzugt sind gram-positive Eubakterien, deren Basenzusammensetzung der DNS einen hohen Guanin- und Cytosinanteil aufweist (G+C Mol %; größer 50 %), insbesondere der Bakterienspezies Micrococcus luteus.

Gegenstand der Erfindung ist somit die Verwendung von Zellwandfraktionen aus gram-positiven Eubakterien für kosmetische Zwecke.

Gegenstand der Erfindung ist ferner ein kosmetisches Mittel, enthaltend Zellwandfraktionen aus gram-positiven Eubakterien, wobei die Zellwandfraktion erhältlich ist durch Aufschluß der Zellen, Abtrennung des Cytoplasmas und enzymatische Zellwandhydrolyse.

Vorteilhaft für die Gewinnung von Zellwandfraktionen aus gram-positiven Bakterien gegenüber solchen aus Pflanzen ist die bessere Reproduzierbarkeit des Ausgangsmaterials durch Einsatz biotechnologischer Prozesse. Weiterhin ist die Separierung bestimmter Zellwandfraktionen aus Bakterien verfahrenstechnisch einfacher, weil aus pflanzlichen Extrakten eine erheblich größere Zahl an unerwünschten Begleitstoffen abgetrennt werden muß.

Pflanzen sind darüber hinaus in ihrer Qualität Einflußfaktoren wie Reifegrad, Bodenbeschaffenheit oder Klima unterworfen.

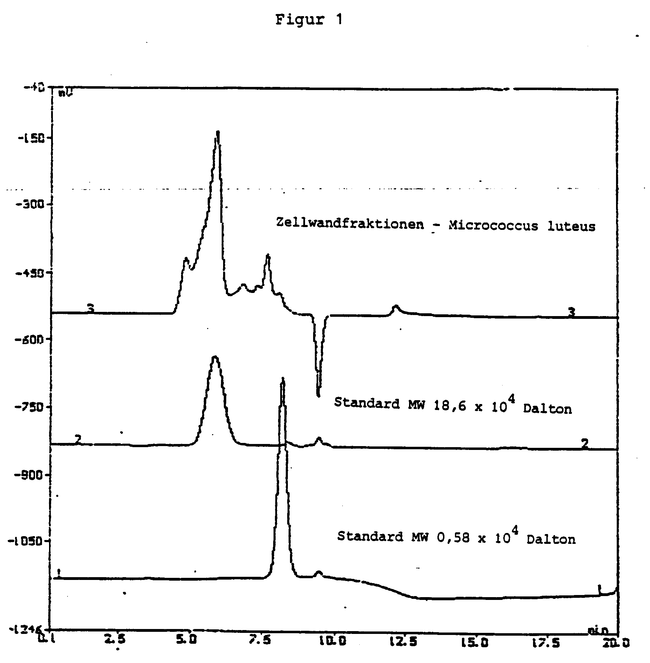

In der beiliegenden Zeichnung zeigt Fig. 1 das Gelpermeationschromatogramm des Zellwandlyophilisats des Micrococcus luteus.

Die grundsätzliche Differenzierung von gram-positiven Eubakterien (nicht zu verwechseln mit der Gattung Eubacterium, die jedoch ebenfalls zu den gram-positiven Eubakterien zählt) in Hoch- und Niedrig-GC-Subdivisionen wurde erst kürzlich etabliert und von Woese et al. phylogenetisch begründet.

(Woese, C.R., Stackebrandt, E., Macke, T.J., and Fox, G.E. 1985. A phylogenetic definition of the major eubacterial taxa. System. Appl. Microbiol. 6:143-151).

Als besonders geeignet hat sich die Gattung Micrococcus und hier wiederum die Spezies Micrococcus luteus erwiesen.

In Bergey's Manual of Systematic Bacteriology, Vol. 2, 1986, S. 1003-1008, werden u.a. die Nährstoffansprüche und die taxonomischen Charakteristika von Micrococcus luteus beschrieben.

Die Zellwand von Micrococcus luteus setzt sich hauptsächlich aus dicken, festen Peptidoglykan-Schichten zusammen. Schleifer und Kandler fanden bei Micrococcen insgesamt sechs verschiedene Peptidoglykan-Typen in der Zellwand.
(Schleifer, K.H. and O. Kandler. 1972. Peptidoglycan types of bacterial cell walls and their taxonomic implications. Bacteriol. Rev. 36: 407-477.)

Teichonsäure fehlt, wohingegen Teichuronsäure nachgewiesen wurde. Yamada, M., A. Hirose und M. Matsuhashi. 1975. Association of lack of cell wall teichuronic acid with formation of cell packets of Micrococcus lysodeikticus (luteus) mutants. J. Bacteriol. 123: 678-686).

Säugetierhaut wird als "primary natural habitat" des Micrococcus luteus angesehen.

Der erfindungsgemäße Wirkstoff im kosmetischen Mittel kann in folgender Weise, z.B. aus der Spezies Micrococcus luteus, hergestellt werden:
Der Micrococcus luteus als Aerobier wird unter gewöhnlichen Bedingungen kultiviert und nach Erreichen der frühstationären Wachstumsphase bei 60 - 65°C für 30 bis 60 Min. pasteurisiert.

Die Zellernte erfolgt mittels Zentrifugation (5 Min., bei 5000 g). Mit steriler physiologischer Kochsalzlösung oder vorzugsweise mit sterilem, neutralen 0,1 molarem Phosphatpuffer wird zwei- bis dreimal gewaschen. Der Zellaufschluß erfolgt unter Einsatz herkömmlicher mechanischer Verfahren, beispielsweise unter Einsatz von Ultraschall, Zellmühle, Hochdruckhomogenisator oder Einsatz der genannten Verfahren in Kombination.

Der Erfolg des Aufschlusses kann im Phasenkontrastmikroskop bei 1000-facher Vergrößerung verfolgt werden.

Die Separierung von Cytoplasma und Zellwand erfolgt durch Zentrifugation (20.000 g für 20 Minuten). Das Sediment wird zweimal mit 0,01 molarem sterilen Phosphatpuffer, pH 7,0, gewaschen. Das Zellwandmaterial wird gefriergetrocknet.

Die Gewinnung aktiver Oligopeptidoglykane (Mureinbestandteile) und Polysaccharide erfolgt mittels enzymatischer Lyse des Rohzellwandmaterials, das mit Proteinen und Nukleinsäuren verunreinigt ist.

Im ersten Schritt der enzymatischen Zellwandhydrolyse werden Proteasen, beispielsweise Trypsin, eingesetzt, um Reste cytoplasmatischer Proteine und Membran-Proteine abzubauen.

Die Suspendierung der lyophilisierten Rohzellwände erfolgt in 50 mM Tris-HCl-Puffer (pH 7,6) unter Einsatz kurzzeitiger Ultraschall-Behandlung. Nach Zusatz von Trypsin erfolgt der Abbau unter konventionellen Bedingungen. Danach wird bei 20.000 g für ca. 20 Minuten zentrifugiert und das Sediment durch mehrfaches Waschen mit obiger Pufferlösung von Trypsin befreit. Die gelartige Konsistenz des Sediments beruht auf Anwesenheit von Nukleotiden, die nach herkömmlichen Methoden durch Nukleasen abgebaut werden können.

Nach Beseitigung der Verunreinigungen erfolgt die Solubilisierung der Zellwände mittels Lysozym. Die Zellwandfraktion wird im 50 mM Tris-HCl-Puffer (pH 7,6) unter Ultrabeschallung suspendiert und Lysozym in erforderlicher Menge zugesetzt. Die Zellwandhydrolyse ist abgeschlossen, wenn kein Transmissionsanstieg der Proben mehr meßbar ist.

Lysozyminaktivierung erfolgt vorzugsweise mittels Proteinase K, die selbst wiederum durch kurzzeitiges Temperieren auf ca. 80°C inaktiviert wird.

Nach Dialyse und Lyophilisierung liegen die Zellwandfraktionen in der gewünschten löslichen Form vor.

Die analytische Charakterisierung des Zellwandlyophilisats erfolgt durch Gelpermeationschromatographie (siehe Fig. 1).
- Säule:: Nucleogel\ GFC 300-8 (Macherey-Nagel)
- Element:: HPLC-Wasser mit 0,02 % NaN₃
- Flow:: 1 ml/min.
- Detektor:: R I
- Polymer-Standard:: Pullulan
(Mp (Dalton) 5.800 - 186.000)
- Ergebnis:: Das lösliche Zellwandmaterial setzt sich aus Fraktionen mit einer mittleren Molekulargewichtsverteilung zwischen 10.000 und 200.000 Dalton zusammen.
(Ausschlußgrenze der Trennsäule bei ca. 300.000 Dalton)

### Wirkungsnachweise:

### In vitro Test an Zellkulturen

Zellwandfraktionen, hergestellt nach dem vorhergehend beschriebenen Verfahren, der folgenden gram-positiven Eubakterien, deren Basenzusammensetzung der DNS durch einen hohen Guanin- und Cytosinanteil (G+C Mol %: größer 50 %) gekennzeichnet ist, wurden beispielhaft für die Testung herangezogen:
Cellulomonas flavigena
Arthrobacter globiformis
Micrococcus luteus.

Das Testziel läßt sich an Makrophagenkulturen realisieren, da Makrophagen im Entzündungsgeschehen eine zentrale Rolle spielen.

Auf vielfache exogene Stimuli reagieren Makrophagen durch Ausschleusung proinflammatorischer Substanzen (endogene Mediatoren), welche sind: Zytokine, Prostaglandine, Leukotriene, Hydroxylinolsäuren und reaktive Sauerstoffspezies.

### Versuchsplan:

Zeitkinetik. Dosisfindung. - Zunächst wurden in Zellkulturen der murinen Makrophagen-Zellinie (J 774) Dosis-Wirkungsbeziehungen und eine Zeitkinetik für die Induktion der Zytokine Tumornekrose-Faktor α (TNF), Interleukin 6 (IL 6) und den Lipidmediator Prostacyklin (PGI₂) ermittelt. Kulturen wurden mit Dosen zwischen 1 µg und 1 mg Trockenmasse pro ml (log Verdünnungen) für 1, 2, 4 und 24 h inkubiert und in den Überständen wurde TNF (Bioassay), IL 6 (Bioassay) und PGI₂ (RIA, als 6-keto-PGF_{2α}) bestimmt.

Der Versuch wurde in Mikrotiterplatten in Triplikaten getrennt für die TNF/IL 6 Bestimmung bzw. PGI₂ Bestimmung durchgeführt.

Hemmung der Bildung von Mediatoren. - J774-Zellkulturen wurden mit Endotoxin (Lipopolysaccharid, LPS) in Gegenwart der Zellwandfraktionen stimuliert.

Danach wurde der Gehalt an TNF im Überstand bestimmt. Die Zellwandfraktionen wurden wiederum in Konzentrationen zwischen 1 µg und 1 mg Trockenmasse pro ml (log Verdünnungen) für 0, 1, 2, 4 und 24 h vorinkubiert, mit LPS stimuliert und in den Überständen wurde TNF/IL 6 bestimmt.
Der Versuch wurde in Mikrotiterplatten in Triplikaten durchgeführt. Auch hier wurden getrennte Ansätze für die Wirkung auf die Synthese von PGI₂ durchgeführt.
Die beste antiinflammatorische Aktivität zeigten überraschend die aus Micrococcus luteus hergestellten Zellwandfraktionen.

Es wurde gleichzeitig an Makrophagen geprüft, ob von den aktiven Zellwandfraktionen von sich aus Entzündungsreaktionen induziert werden. Als Ergebnis zeigte sich, daß nach Einwirkung von Zellwandfraktionen aus Micrococcus luteus keine Zytokine ausgeschleust werden, d.h. der erfindungsgemäße Wirkstoff induziert selbst keine proinflammatorischen Reaktionen.

### Beispiele kosmetischer Mittel

Der erfindungsgemäße Wirkstoff - Zellwandfraktionen aus Micrococcus luteus, gelöst in einer schwach sauren Pufferlösung - kann in unterschiedlichsten kosmetischen Darreichungsformen in Kombination mit üblichen kosmetischen Inhalt- und Grundstoffen eingearbeitet werden. Die folgenden Rezepturen der kosmetischen Mittel stellen eine exemplarische Auswahl dar.

### Beispiel 1

| Creme O/W | | |
|---|---|---|
| a) | Gemisch aus Partialglyceriden, Fettalkoholen Wachsestern und ethoxylierten Fettalkoholen (Emulgade® SE) | 3 % |
| | Capryl-Caprinsäureester gesättigter Fettalkohole (Cetiol® LC) | 5 % |
| | Partialglyceride und Ester langkettiger Fettsäuren (Cutina® BW) | 2 % |
| | Stearylalkohol | 3 % |
| | Konservierungsmittel | q.s. |
| b) | Wasser, destilliert | 71,9 % |
| | Konservierungsmittel | q.s. |
| | Glycerin | 3,0 % |
| | Polyacrylat (Carbopol® 954) | 0,3 % |
| | Kaliumhydroxid (10%-ig) | 1,2 % |
| c) | Zellwandfraktionen | 10,0 % |

### Beispiel 2

| Creme W/O | | |
|---|---|---|
| a) | Polysilixan-polyalkyl-polyether-Copolymer (Abil® EM 90) | 2,5 % |
| | Oleyl-Erucat (Cetiol® I 600) | 8,0 % |
| | Isopropylmyristat | 14,0 % |
| | Bienenwachs | 5,0 % |
| | Paraffinum subliquidum | 5,0 % |
| | Stearylalkohol | 5,0 % |
| | Konservierungsmittel | q.s. |
| b) | Wasser, destilliert | 44,4 % |
| | Konservierungsmittel | q.s. |
| | Glycerin | 5,0 % |
| | Natriumchlorid | 0,5 % |
| c) | Zellwandfraktionen | 10,0 % |

### Beispiel 3

| Gel | | |
|---|---|---|
| a) | Glycerin Polyacrylat (Hispagel® 200) | 20,0 % |
| | Xanthan Gum (1%ig) | 30,0 % |
| | Wasser, destilliert | 35,7 % |
| | Konservierungsmittel | q.s. |
| | Oleyl-Erucat (Cetiol® I 600) | 4,0 % |
| b) | Zellwandfraktionen | 10,0 % |

## Patentansprüche

1. Verwendung von Zellwandfraktionen aus gram-positiven Eubakterien für kosmetische Zwecke.

2. Kosmetisches Mittel, enthaltend Zellwandfraktionen aus gram-positiven Eubakterien, wobei die Zellwandfraktion erhältlich ist durch Aufschluß der Zellen, Abtrennung des Cytoplasmas und enzymatische Zellwandhydrolyse.

3. Verwendung gemäß Anspruch 1, wobei die DNS-Basenzusammensetzung der gram-positiven Eubakterien einen Guanin- und Cytosinanteil (G + C Mol %) größer 50 % aufweist.

4. Verwendung gemäß einem der Ansprüche 1 oder 3, wobei der verwendete Bakterienstamm nicht-pathogen ist.

5. Verwendung gemäß einem der Ansprüche 1, 3 oder 4, wobei der verwendete Bakterienstamm der Spezies Micrococcus luteus oder Cellulomonas flavigena oder Arthrobacter globiformis angehört.

6. Kosmetisches Mittel gemäß Anspruch 2, wobei die DNA-Basenzusammensetzung des verwendeten Bakterienstammes einen Guanin- und Cytosinanteil (G + C Mol %) größer 50% aufweist.

7. Kosmetisches Mittel gemäß einem der Ansprüche 2 oder 6, wobei der verwendete Bakterienstamm nicht-pathogen ist.

8. Kosmetisches Mittel gemäß einem der Ansprüche 2, 6 oder 7, wobei der verwendete Bakterienstamm der Spezies Micrococcus luteus oder Cellulomonas flavigena oder Arthrobacter globiformis angehört.

9. Kosmetisches Mittel gemäß einem der Ansprüche 2, 6, 7 oder 8, wobei die enzymatische Zellwandhydrolyse unter Verwendung von Trypsin und Lysozym erfolgt.

10. Kosmetisches Mittel, enthaltend Zellwandfraktionen aus Micrococcus luteus oder Cellulomonas flavigena oder Arthrobacter globiformis.

## Claims

1. The use of cell wall fractions from gram positive Eubacteria for cosmetic purposes.

2. A cosmetic composition containing cell wall fractions of gram positive Eubacteria, the cell wall fraction being obtainable by cell disruption, separation of the cytoplasm and enzymatic cell wall hydrolysis.

3. The use according to claim 1, wherein the DNA base composition of the gram positive Eubacteria has a guanine and cytosine content (G + C mole %) which is greater than 50%.

4. The use according to any one of claims 1 or 3, wherein the bacterial strain that is used is non-pathogenic.

5. The use according to any one of claims 1, 3 or 4, wherein the bacterial strain that is used belongs to the species Micrococcus luteus or Cellulomonas flavigena or Arthrobacter globiformis.

6. The cosmetic composition according to claim 2, wherein the DNA base composition of the bacterial strain that is used has guanine and cytosine portions (G + C mole %) greater than 50%.

7. The cosmetic composition according any one of claims 2 or 6, wherein the bacterial strain that is used is non-pathogenic.

8. The cosmetic composition according to any one of claims 2, 6 or 7 wherein the bacterial strain that is used belongs to the species Micrococcus luteus or Cellulomonas flavigena or Arthrobacter globiformis.

9. The cosmetic composition according to any one of claims 2, 6, 7 or 8, wherein the enzymatic cell wall hydrolysis is performed with the use of trypsin and lysozyme.

10. A cosmetic composition containing cell wall fractions of Micrococcus luteus or Cellulomonas flavigena or Arthrobacter globiformis.

## Revendications

1. Utilisation de fractions de parois cellulaires provenant d'eubactéries Gram positif dans des buts cosmétiques.

2. Produits cosmétiques, contenant des fractions de parois cellulaires provenant d'eubactéries Gram positif, la fraction de parois cellulaires étant préparable par désintégration des cellules, isolement du cytoplasme et hydrolyse enzymatique de la paroi cellulaire.

3. Utilisation suivant la revendication 1, dans laquelle la composition des bases d'ADN des eubactéries Gram positif présente une fraction de guanine et de cytosine (moles % de G + C) supérieure à 50 %.

4. Utilisation suivant l'une des revendications 1 et 3, dans laquelle la souche utilisée de bactéries n'est pas pathogène.

5. Utilisation suivant l'une des revendications 1, 3 et 4, dans laquelle la souche utilisée de bactéries appartient à l'espèce Micrococcus luteus ou Cellulomonas flavigena ou Arthrobacter globiformis.

6. Produits cosmétiques suivant la revendication 2, caractérisés en ce que la composition des bases d'ADN de la souche utilisée de bactéries présente une fraction de guanine et de cytosine (moles % de G + C) supérieure à 50 %.

7. Produits cosmétiques suivant l'une des revendications 2 et 6, caractérisés en ce que la souche utilisée de bactéries n'est pas pathogène.

8. Produits cosmétiques suivant l'une des revendications 2, 6 et 7, caractérisés en ce que la souche utilisée de bactéries appartient à l'espèce Micrococcus luteus ou Cellulomonas flavigena ou Arthrobacter globiformis.

9. Produits cosmétiques suivant l'une des revendications 2, 6, 7 et 8, caractérisés en ce que l'hydrolyse enzymatique des parois cellulaires est effectuée avec utilisation de trypsine et de lysozyme.

10. Produits cosmétiques, contenant des fractions de parois cellulaires provenant de Micrococcus luteus ou Cellulomonas flavigena ou Arthrobacter globiformis.
